(19) Europäisches Patentamt

European Patent Office

Office européen des brevets

(11) **EP 4 681 794 A1**

(12) **EUROPEAN PATENT APPLICATION**
published in accordance with Art. 153(4) EPC

(43) Date of publication:
**21.01.2026 Bulletin 2026/04**

(21) Application number: **24846667.4**

(22) Date of filing: **25.06.2024**

(51) International Patent Classification (IPC):
*B01D 3/32* (2006.01)         *B01D 3/22* (2006.01)
*B01D 3/42* (2006.01)         *C07C 51/44* (2006.01)
*C07C 67/54* (2006.01)         *C07C 7/04* (2006.01)
*C07C 57/04* (2006.01)         *C07C 69/54* (2006.01)
*C07C 11/167* (2006.01)

(52) Cooperative Patent Classification (CPC):
**B01D 3/22; B01D 3/32; B01D 3/42; C07C 7/04;
C07C 11/167; C07C 51/44; C07C 57/04;
C07C 67/54; C07C 69/54**

(86) International application number:
**PCT/KR2024/008754**

(87) International publication number:
**WO 2025/063435 (27.03.2025 Gazette 2025/13)**

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**GE KH MA MD TN**

(30) Priority: **19.09.2023 KR 20230124652**

(71) Applicant: **LG Chem, Ltd.**
**Seoul 07336 (KR)**

(72) Inventors:
• **OH, Jai June**
  **Daejeon 34122 (KR)**
• **JANG, Kyung Soo**
  **Daejeon 34122 (KR)**
• **LEE, Sung Kyu**
  **Daejeon 34122 (KR)**

(74) Representative: **Goddar, Heinz J.**
**Boehmert & Boehmert
Anwaltspartnerschaft mbB
Pettenkoferstrasse 22
80336 München (DE)**

(54) **REACTIVE MONOMER SEPARATION APPARATUS**

(57)     The present disclosure relates to a reactive monomer separation device, and more specifically, to a reactive monomer separation device in which a structure of a feed supply unit is designed so that feed can be uniformly distributed inside a column, thereby improving column efficiency.

FIG. 2

## Description

Cross Reference to Related Applications

This application claims priority to and the benefit of Korean Patent Application No. 10-2023-0124652 filed on September 19, 2023, which is hereby incorporated by reference in their entirety.

Technical Field

The present disclosure relates to a reactive monomer separation device, and more specifically, to a reactive monomer separation device in which a structure of a feed supply unit is designed so that feed can be uniformly distributed inside a column, thereby improving column efficiency.

## Background Art

A dual flow tray is a distillation device designed so that gas and liquid move up and down through a hole and make contact without a downcomer. Specifically, the dual flow tray is used in a case where liquid flow occurs due to weeping liquid in the hole of the tray since the downcomer is not provided, and a substance to be separated, for example, a reactive monomer, is severely polymerized inside the column to form a polymer, and fouling is a concern.

The dual flow tray has the advantage of a large allowable flow rate and can be used to respond to polymer fouling, but has the disadvantage of being lower in efficiency than columns using other types of existing trays due to a channeling phenomenon. The channeling phenomenon is the main factor that reduces the efficiency of a column, and the larger the diameter of the column, the more likely it is that the channeling phenomenon occurs.

Specifically, the channeling phenomenon is a phenomenon in which the liquid and gas flow is unevenly distributed due to physical factors such as uneven distribution of feed or tray blockage, and when this channeling phenomenon occurs, the gas-liquid contact area decreases, and as a result, the gas-liquid contact for each stage occurs in a smaller region compared to the design capacity. In addition, since there is no downcomer in the dual flow tray, the liquid redistribution does not occur, and as a result, the overall efficiency of the column decreases, which causes problems such as a decrease in product quality and an increase in energy consumption.

In this way, since the uneven distribution of feed in the column using the dual flow tray generates the channeling phenomenon, even distribution of feed is one of the factors that determines the efficiency of the column, but in the related art, even distribution of feed within the column is designed without being considered, and the column is operated with low efficiency. Therefore, it is necessary to develop a design for a feed supply structure that enables even distribution of feed in the column using the dual flow tray.

## Brief Description

## Technical Problem

The problem to be solved by the present disclosure is to solve the problems mentioned in the Background technology of the above invention, and an object of the present disclosure is to provide a reactive monomer separation device with improved column efficiency by designing a structure of a feed supply unit so that the feed can be uniformly distributed within the column in order to solve the problem mentioned in the technology that is the background of the above disclosure.

However, an object of the present disclosure is not limited to the object mentioned above, and other objects that are not mentioned can be clearly understood by those skilled in the art from the description below.

## Technical Solution

According to an embodiment of the present disclosure, there is provided a reactive monomer separation device including: a cylindrical column; a plurality of dual flow trays provided inside the cylindrical column to partition a plurality of stages; and a feed supply unit provided in one of the plurality of stages to supply a raw material including a liquid reactive monomer.

Moreover, the feed supply unit may include a circular feed transfer pipe provided along an inner wall of the cylindrical column, one or more inner pipes extending in a direction of a central axis from the circular feed transfer pipe, and a spray nozzle provided at each end of each of the one or more inner pipes.

A spray angle ($\theta$) of the spray nozzle may be equal to or less than a maximum spray angle defined by the following Equation (1).

$$\text{Equation (1): maximum spray angle} = 2 \times \tan^{-1}(l/h)$$

In the Equation (1), l is a length of the inner pipe, and h is a height from a lower dual flow tray of the stage equipped with the feed supply unit to the feed supply unit.

**Advantageous Effects**

According to the reactive monomer separation device of the present disclosure, by designing the structure of the feed supply unit to improve an area of a feed distribution region of the feed stage in the dual flow tray, a channeling phenomenon can be prevented, and a gas-liquid contact area can be maximized to increase the efficiency of the entire column.

In addition, by improving the structure of the feed supply unit so that the feed can be distributed uniformly and widely in the dual flow tray, the channeling phenomenon can be prevented, and the deterioration of product quality and the increase in energy usage due to the decrease in the overall efficiency of the column can be prevented.

The effects that can be obtained in the present specification are not limited to the effects mentioned above, and other effects that are not mentioned can be clearly understood by those skilled in the art to which the present disclosure belongs from the description below.

**BRIEF DESCRIPTION OF THE DRAWINGS**

FIG. 1 is a cross-sectional view of a separation device according to an embodiment of the present disclosure.
FIG. 2 is a perspective view illustrating in detail an enlarged region A of FIG. 1.
FIG. 3 is a view illustrating what symbols used in Equations (1) and (2) mean.
FIG. 4 is a side view illustrating a case where the feed supply unit is positioned below a feed solution level on a lower dual flow tray as a comparative example.
FIG. 5 is a side view illustrating a spray region in a case where the feed supply unit is positioned too high when a length of an inner pipe is short and a spray angle of a spray nozzle is large as a comparative example.
FIG. 6 is a top view illustrating a minimum feed spray region B when a minimum spray angle is satisfied in a case where the number of spray nozzles is 4 and the length of the inner pipe is long as an embodiment of the present disclosure.
FIG. 7 is a top view illustrating a maximum feed spray region B when the maximum spray angle is satisfied in a case where the number of spray nozzles is 4 and the length of the inner pipe is long as an embodiment of the present disclosure.
FIG. 8 is a top view illustrating an optimal feed spray region B when an optimal spray angle is satisfied in a case where the number of spray nozzles is 4 and the length of the inner pipe is short as an embodiment of the present disclosure.
FIG. 9 is a top view illustrating the feed spray region B when the maximum spray angle is exceeded in a case where the number of spray nozzles is 4 and the length of the inner pipe is short as a comparative example.
FIG. 10 illustrates a radius of each of the minimum and maximum feed spray region according to the inner pipe length.
FIG. 11 is a side view illustrating the radius of the feed spray region according to the spray angle and height of the spray nozzle.
FIG. 12 is a graph illustrating the maximum and minimum radius of the feed spray region according to the inner pipe length.
FIG. 13 is a graph illustrating the minimum and maximum spray angle according to the inner pipe length when the number of spray nozzles is 4 in an embodiment of the present disclosure.
FIG. 14 is a graph illustrating the minimum and maximum spray angle according to the number of spray nozzles according to the inner pipe length in an embodiment of the present disclosure.

**Detailed Description**

Terms or words used in the description and claims of the present disclosure should not be interpreted as limited to their usual or dictionary meanings, and should be interpreted as meanings and concepts that conform to the technical idea of the present disclosure based on the principle that the inventor can appropriately define the concept of the term in order to explain his own disclosure in the best way.

In relation to the description of the drawings, similar reference numerals may be used for similar or related components.

The singular form of a noun corresponding to an item may include one or more of the items, unless the relevant context clearly indicates otherwise.

In the present disclosure, each of the phrases such as "A or B", "at least one of A and B", "at least one of A or B", "A, B or C", "at least one of A, B and C", and "at least one of A, B, or C" may include any one of the items listed together in the corresponding phrase, or all possible combinations thereof.

The term "and/or" includes a combination of multiple related described components or any one of multiple related described components.

Terms such as "first," "second," or "first" or "second" may be used simply to distinguish the corresponding component from other corresponding components and do not limit the corresponding components in any other aspect (for example, importance or order).

In addition, terms such as "front surface", "back surface", "upper surface", "lower surface", "side surface", "left", "right", "upper portion", and "lower portion" used herein are defined based on the drawings, and the shape and position of each component are not limited by these terms.

Terms such as "include" or "have" are intended to specify the presence of a feature, number, step, operation, component, part, or combination thereof described in the present disclosure, but do not preclude the presence or addition of one or more other features, numbers, steps, operations, components, parts, or combinations thereof.

When a component is said to be "connected", "coupled" "supported", or "contacted" with another component, this includes not only cases where the components are directly connected, coupled, supported, or contacted, but also cases where the components are indirectly connected, coupled, supported, or contacted through a third component.

When a component is said to be "on" another component, this includes not only cases where the component is in contact with the other component, but also cases where another component exists between the two components.

In addition, as used herein, the terms "about" and "substantially" are used to mean at or near the numerical value when a unique manufacturing and material tolerance is presented, and are used to prevent unscrupulous infringers from unfairly utilizing the disclosure in which exact or absolute values are mentioned to help understanding of the present disclosure.

The term "stream" used herein may mean the flow of fluid within a process, and may also mean the fluid itself flowing within a pipe. Specifically, the stream may mean both the fluid itself flowing within a pipe connecting each device, and the flow of the fluid. In addition, the fluid may include at least one component of gas, liquid, and solid.

In the present disclosure, a "bottom" of the device means, unless otherwise specified, a point at a height of 95% to 100% downward from the uppermost portion of the device, and may specifically mean the lowest part. Similarly, a "top" of the device means, unless otherwise specified, a point at a height of 0% to 5% downward from the uppermost portion of the device, and may specifically mean the highest part.

In the present specification, a "depth of a spray nozzle" means a distance separated from the inside of a column 100 located most adjacent to a spray nozzle 330, and may be used with the same meaning as a "length 1 of an inner pipe," and a "height of the spray nozzle" or a "height of a feed supply unit" may mean a height h from a lower tray 210a to the feed supply unit (or spray nozzle). In addition, a "tray spacing TS" may mean the height from the lower tray 210a to an upper tray 210b in the feed stage.

Hereinafter, the reactive monomer separation device according to the present disclosure will be described in detail with reference to the drawings.

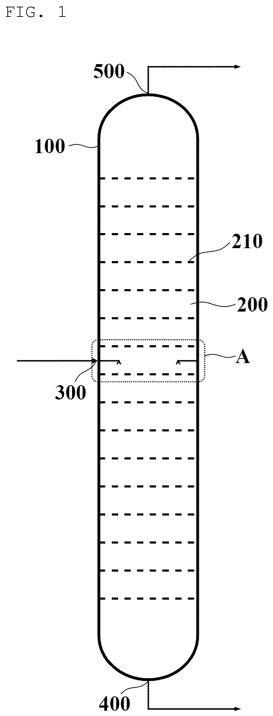

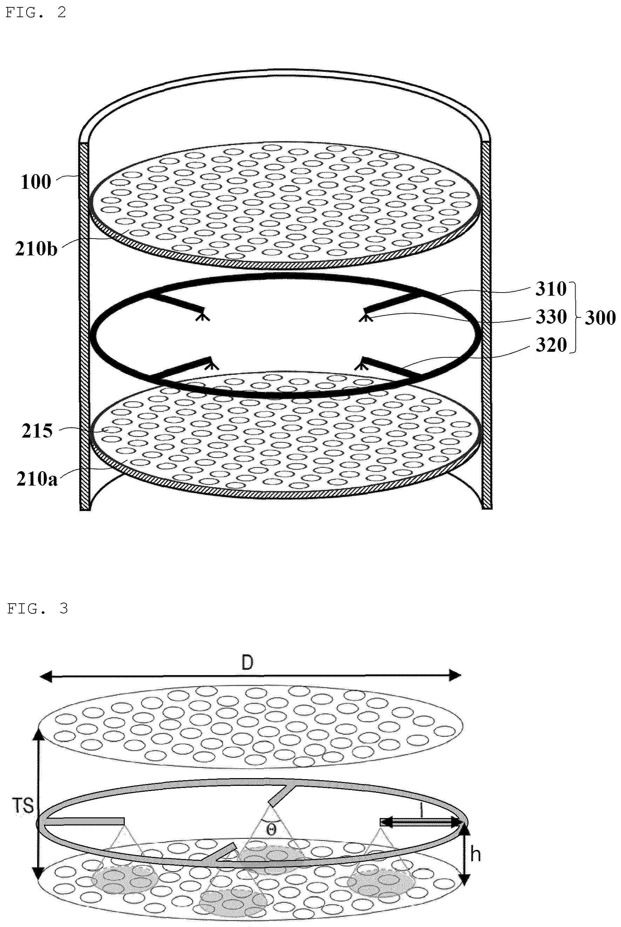

FIG. 1 is a cross-sectional view of the reactive monomer separation device according to an embodiment, and FIG. 2 is a perspective view illustrating an enlarged region A of FIG. 1, and more specifically, a detailed structural diagram of a feed stage A equipped with a feed supply unit 300.

Referring to FIG. 1, the reactive monomer separation device according to an embodiment of the present disclosure includes a cylindrical column 100.

The cylindrical column 100 can be applied without limitation as long as it is a form generally used in a separation process through gas-liquid contact, and the size of the cylindrical column 100 can be appropriately selected within a range commonly applied in the relevant field, and there is no special limitation. For example, an inner diameter of the cylindrical column 100 may be about 300 mm to 6,000 mm, specifically about 1,500 mm to 6,000 mm, more specifically about 1,800 mm to 4,000 mm, and the overall height of the cylindrical column 100 may be about 2,000 mm to 60,000 mm, specifically about 5,000 mm to 50,000 mm, more specifically about 6,000 mm to 40,000 mm, but is not limited thereto.

In addition, the reactive monomer separation device according to an embodiment of the present disclosure is provided with a plurality of dual flow trays 210 inside the cylindrical column 100. The reactive monomer, which is a separation target substance, can be polymerized inside the column to form a polymer, which increases the risk of fouling. Therefore, in the present disclosure, the dual flow tray 210 is used as a tray that divides the stage of a distillation column for gas-liquid separation.

The dual flow tray 210 is a sieve tray without a downcomer, and performs a dual function of allowing both liquid and vapor to pass through a hole 215 of the dual flow tray. In addition, since the dual flow tray does not have a downcomer, a larger tray region is provided, so it has the advantage of having a larger capacity and being easy to install and maintain than a general tray type. However, since the dual flow tray operates in a state where the liquid continuously flows through the hole 215, it has low efficiency and is susceptible to channeling, making it sensitive to the liquid distribution of the feed. Accordingly, in the present disclosure, the structure of the feed supply unit 300 described below is designed so that the liquid feed can be distributed as evenly as possible in order to improve the separation efficiency of the dual flow tray and prevent the occurrence of channeling.

As illustrated in FIG. 1, the plurality of dual flow trays 210 are arranged in a vertical direction with respect to the height direction of the cylindrical column 100 and can be arranged to be spaced apart by a predetermined interval TS. The interior of the cylindrical column 100 can be divided into a plurality of stages 200 by the plurality of dual flow trays 210.

The number and size of the dual flow trays 210 are not particularly limited and can be set based on the number of theoretical stages inferred from a distillation curve considering the composition of the feed stream. In the above, the "number of theoretical stages" means a virtual region in which two phases, such as gas and liquid, are in equilibrium with each other in a separation device or the number of stages divided by a plurality of trays.

According to an embodiment of the present disclosure, the feed supply unit 300 is provided in one of the plurality of stages 200, that is, a predetermined feed stage A, so as to supply a liquid raw material into the column. In this case, the raw material may include one or more reactive monomers, or a mixture containing them. Examples of the reactive monomers include, but are not limited to, (meth)acrylic acid, acrylic acid, methyl acrylate, and 1,3-butadiene.

Referring to FIG. 2, the feed supply unit 300 may be spaced apart from the lower dual flow tray 210a and the upper dual flow tray 210b in the predetermined feed stage A. The height h of the feed supply unit 300 may be determined according to the tray spacing TS, the column inner diameter, a depth 1 of the spray nozzle to be described later, and/or a spray angle $\theta$. For example, considering that the area of the feed injection area increases as the feed supply unit 300 is installed higher, it may be preferable that the feed supply unit 300 be installed closer to the upper dual flow tray 210b than to the lower dual flow tray 210a, but it is not limited thereto. For example, the height h of the feed supply unit may be more than 0 mm and less than TS, and more specifically, more than TS/2 and less than TS. However, it is preferable that the feed supply unit 300 is installed at a height that does not contact the upper dual flow tray 210b but can be spaced apart from the upper dual flow tray 210b.

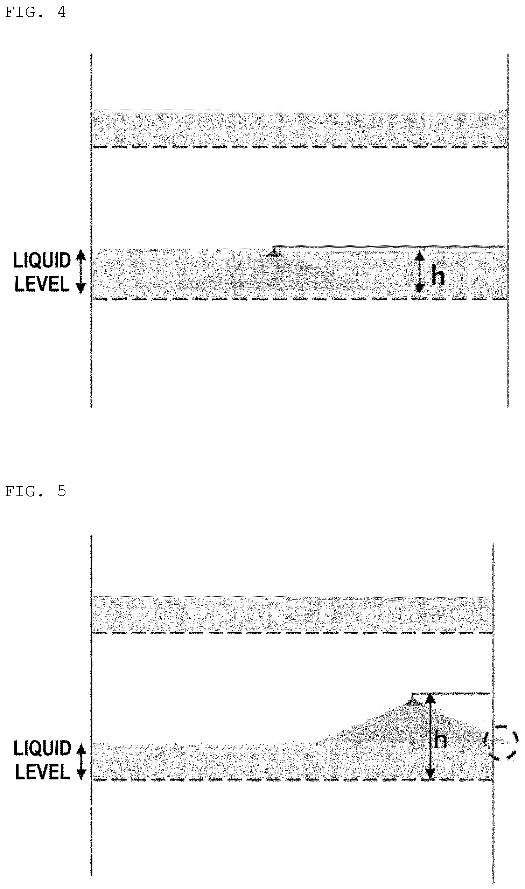

For example, when the feed supply unit 300 is installed too low, as illustrated in FIG. 4, the spray nozzle may be positioned lower than the feed solution level (liquid level) on the lower dual flow tray. In this case, the solution level may be calculated using a simulation program, or the like. In addition, in a case the length 1 of the inner pipe is short and the spray angle $\theta$ of the spray nozzle is large, when the feed supply unit 300 is installed too high, as illustrated in FIG. 5, the radius of the feed injection area becomes large, causing the sprayed feed to flow down the inner wall of the column, thereby reducing the efficiency of the column.

In the reactive monomer separation device according to an embodiment of the present disclosure, the feed supply unit 300 includes a feed transfer pipe 310, an inner pipe 320, and a spray nozzle 330. The reactive monomer is a material that spontaneously forms a polymer. Therefore, when the reactive monomer is supplied to a separation device as a feed, a liquid distributor used in a general column cannot be used because it is prone to polymer fouling due to the complex structure of the liquid distributer. Therefore, in the reactive monomer separation device according to the present disclosure, the spray nozzle 330 having a relatively simple structure is used to reduce the possibility of polymer fouling, and the feed containing the reactive monomer is distributed as uniformly as possible on the tray of the feed stage.

Referring to FIG. 2, the feed transfer pipe 310 may be provided in a circular shape along the inner wall of the cylindrical column 100. Through the structure as described above, interference of the solution descending from the upper tray 210b may be minimized.

In an embodiment, the inner pipe 320 may be extended from the circular feed transfer pipe 310 to have a predetermined length 1 in the center axis direction. For example, the length 1 of the inner pipe 320 may be less than 0.5D, specifically more than 0 mm and less than 0.5D, and more specifically 0.15D to 0.35D or less. Here, D is the inner diameter of the cylindrical column (see FIG. 3). The closer the length l of the inner pipe is to 0.5D, the larger the maximum spray angle becomes, which maximizes the feed spray region. However, the area of the feed spray region may not significantly increase due to physical constraints caused by the spray angle limitations of commercial nozzles. In addition, when two or more nozzles are used, the longer the length l of the inner pipe, the larger the overall area of the feed spray region may become, but overlap between the feed spray regions may occur, which may not significantly increase the feed distribution efficiency. Meanwhile, when the inner pipe 320 is absent or the length of the inner pipe is too short, the feed spray region may be limited, making efficient feed distribution difficult.

The inner pipe 320 may be provided as one or more, and two or more are preferable. The number of the inner pipes 320 may be appropriately selected in consideration of column efficiency. For example, the number of the inner pipes may be 2 to 10, specifically 2 to 8, more specifically 4 to 6.

In addition, the one or more inner pipes 320 may be provided spaced apart from each other at equal intervals. When a plurality of inner pipes 320 are arranged spaced apart from each other at equal intervals, the feed spray regions can be more uniformly distributed by reducing an overlapping region in which the feed spray regions overlap each other and a non-spray region.

In an embodiment, the spray nozzle 330 is provided at each end of each of the one or more inner pipes 320. The number n of the spray nozzles may correspond 1:1 to the number of the inner pipes. For example, the number n of the spray nozzles may be 2 to 10, specifically 2 to 8, more specifically 4 to 6.

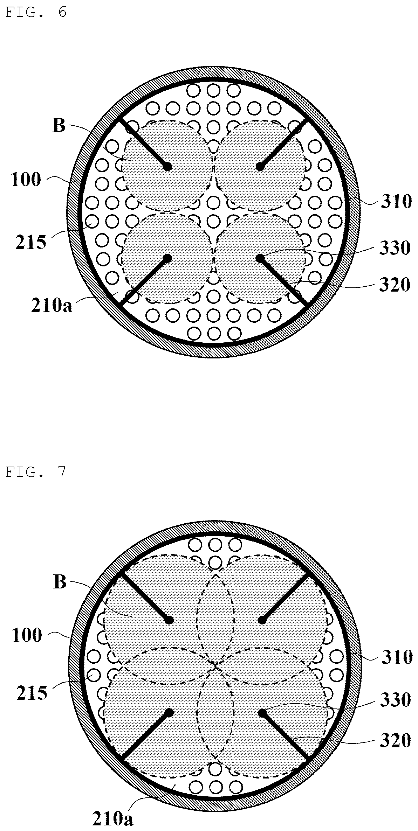

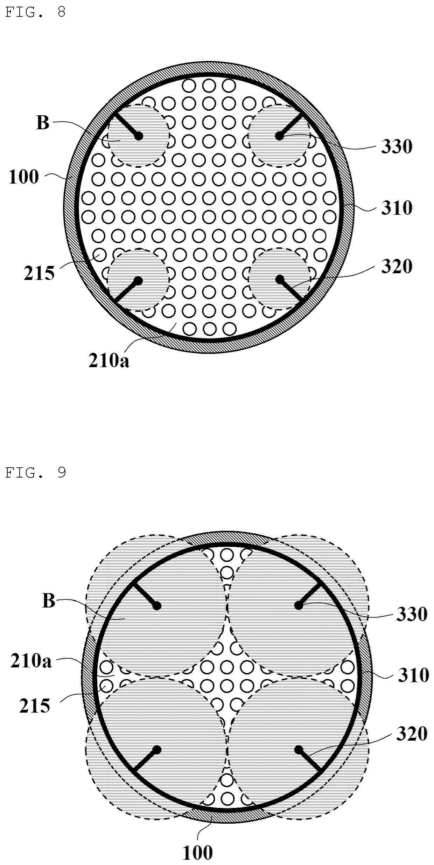

The spray angle $\theta$ of the above spray nozzle can be adjusted by considering the inner diameter D of the column, the length l of the inner pipe, the height h of the feed supply unit, and/or the number n of the spray nozzles. FIGS. 6 to 9 illustrate a feed spray region B according to the length of the inner pipe 320 and the spray angle of the spray nozzle when the number of spray nozzles 330 is 4. Specifically, FIGS. 6 and 7 illustrate a minimum feed spray region when the length of the inner pipe 320 is long and the spray nozzle 330 satisfies the minimum spray angle and a maximum feed spray region when the spray nozzle 330 satisfies the maximum spray angle, respectively. Meanwhile, FIGS. 8 and 9 illustrate an optimal feed spray region when the length of the inner pipe 320 is short, the spray nozzle 330 satisfies the optimal spray angle, and a feed spray region when the spray nozzle 330 exceeds the optimal spray angle, respectively.

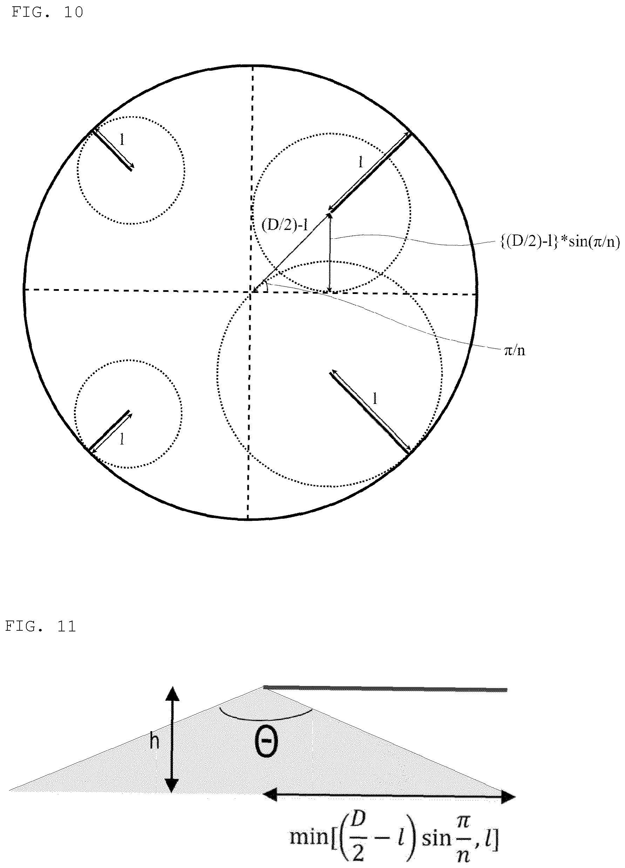

More specifically, in a case where the number n of the spray nozzles 330 is 4 and the length 1 of the inner pipe 320 is longer than a certain level, when the spray nozzle 330 satisfies the minimum spray angle, the area of the overall feed spray region B is somewhat reduced as illustrated in FIG. 6, but the overlapping feed spray regions can be minimized. More specifically, referring to the upper right of FIG. 10, when the spray nozzle 330 satisfies the minimum spray angle, a radius $[\{(D/2)-1\}*\sin(\pi/n)]$ of the feed spray region B can be smaller than the length l of the inner pipe.

In addition, in a case where the number n of spray nozzles 330 is 4 and the length 1 of the inner pipe 320 is longer than a certain level, when the maximum spray angle is satisfied, the area of the entire feed spray region B can be maximized as illustrated in FIG. 7, but some feed spray regions that overlap each other exist. Referring to the lower right end of FIG. 10, when the spray nozzle satisfies the maximum spray angle, the length 1 of the inner pipe and the radius of the feed spray region may be the same.

Meanwhile, as illustrated in FIG. 8, in a case where the number n of spray nozzles 330 is 4 and the length 1 of the inner pipe 320 is shorter than a certain level, even when the feed spray region B is sprayed at the maximum spray angle of the spray nozzle 330 that does not touch the inner wall of the column 100, there is no overlapping feed spray region B, and thus, the minimum spray angle and the maximum spray angle of the spray nozzle 330 may be the same (see the left side of FIG. 10). In this case, when the minimum spray angle and the maximum spray angle of the spray nozzle 330 are the same, the optimum spray angle can be achieved.

Meanwhile, in a case where the number n of spray nozzles 330 is 4 and the length l of the inner pipe 320 is shorter than a certain level, as illustrated in FIG. 9, when the spray nozzle 330 exceeds the optimal spray angle in order to expand the area of the feed spray region B, the sprayed feed flows down the inner wall of the column 100, which causes a problem in that the column efficiency is reduced.

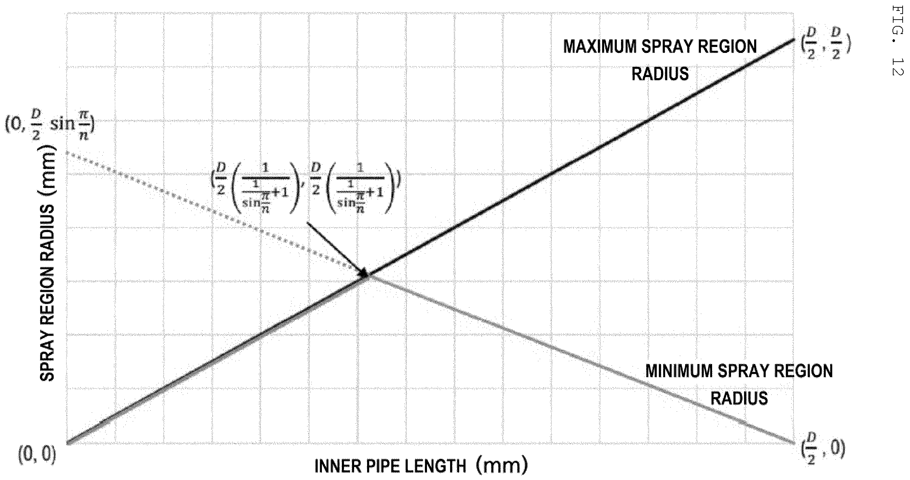

In FIG. 10, the left side illustrates the minimum and maximum feed spray regions when the inner pipe length 1 is short, the right side illustrates the minimum and maximum feed spray regions when the inner pipe length 1 is long, and FIG. 11 illustrates the radius of the feed spray region B according to the spray angle $\theta$ and height h of the spray nozzle. In addition, FIG. 12 is a graph illustrating the maximum and minimum radii of the feed spray region according to the inner pipe length. In this way, the maximum and minimum spray angles of the spray nozzle can be derived by referring to FIGS. 10 to 12.

When all conditions are fixed except the spray angle $\theta$ of the spray nozzle, the area of the feed spray region increases as the spray angle of the spray nozzle increases. However, when the sprayed feed comes into contact with the column inner wall, the feed flows down the column inner wall, and since this feed solution does not undergo gas-liquid separation, it is necessary to adjust the spray angle of the spray nozzle so that the feed does not come into contact with the column inner wall.

Accordingly, it is preferable that the spray angle $\theta$ of the spray nozzle 330 is adjusted to be less than the maximum spray angle defined by the following Equation (1).

$$\text{Equation (1): maximum spray angle} = 2 \times \tan^{-1}(l/h)$$

In the above Equation (1), 1 is the length of the inner pipe, and h is the height from the lower dual flow tray of the stage equipped with the feed supply unit to the feed supply unit (see FIG. 3).

Equation (1) above is the maximum spray angle that can maximize the area of the entire feed spray region while the feed sprayed from the spray nozzle does not come into contact with the column inner wall. That is, when the spray angle $\theta$ of the spray nozzle satisfies Equation (1), as illustrated in FIGS. 7 and 8, the area of the feed spray region can be maximized, while preventing the problem of the feed sprayed from the spray nozzle flowing down the column inner wall and reducing column efficiency due to excessive expansion of the feed spray region B as illustrated in FIG. 9.

Furthermore, referring to FIGS. 10 and 11, considering the feed distribution efficiency, the minimum radius of the feed spray region that can minimize the region where the feed spray regions overlap while maximizing the total feed spray region can be selected according to the length of the inner pipe. Specifically, the minimum radius of the feed spray region can be a smaller value between the length of the inner pipe and the value calculated by $[\{(D/2)-l\}*\sin(\pi/n)]$.

Accordingly, it is preferable that the spray angle θ of the spray nozzle be adjusted to an angle more than the minimum spray angle defined by Equation (2) below.

$$\text{minimum spray angle} = 2 \times \tan^{-1}(\min[\{(D/2)-l\} \times \sin(\pi/n),l]/h) \hspace{2cm} \text{Equation (2):}$$

In the above Equation (2), D is the inner diameter of the cylindrical column, 1 is the length of the inner pipe, n is the number of spray nozzles, and h is the height from the lower dual flow tray of the stage equipped with the feed supply unit to the feed supply unit (see FIG. 3). Here, the n spray nozzles are spaced apart from each other at the same interval (distance).

The above Equation (2) is the minimum spray angle that can minimize the region where the feed spray regions B overlap each other. In other words, the spray angle θ of the spray nozzle should satisfy the above Equation (2) to minimize the overlap of the feed spray regions while maximizing the area. Referring to FIG. 12, when the length of the inner pipe is equal to or less than a point $[(D/2)*(\sin(\pi/n)^{-1}+1)^{-1}]$ where the radius of the minimum spray region and the radius of the maximum spray region intersect, it can be seen that the radius of the minimum spray region and the radius of the maximum spray region are the same, and therefore the minimum spray angle and the maximum spray angle of the spray nozzle are also the same.

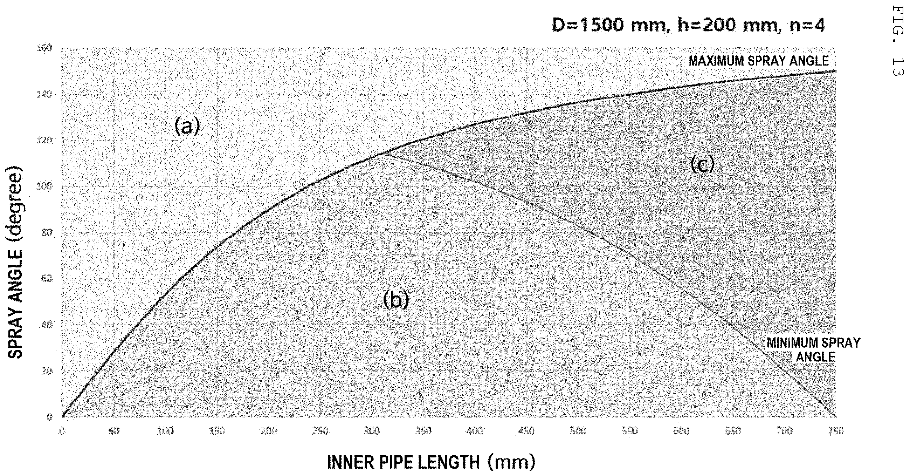

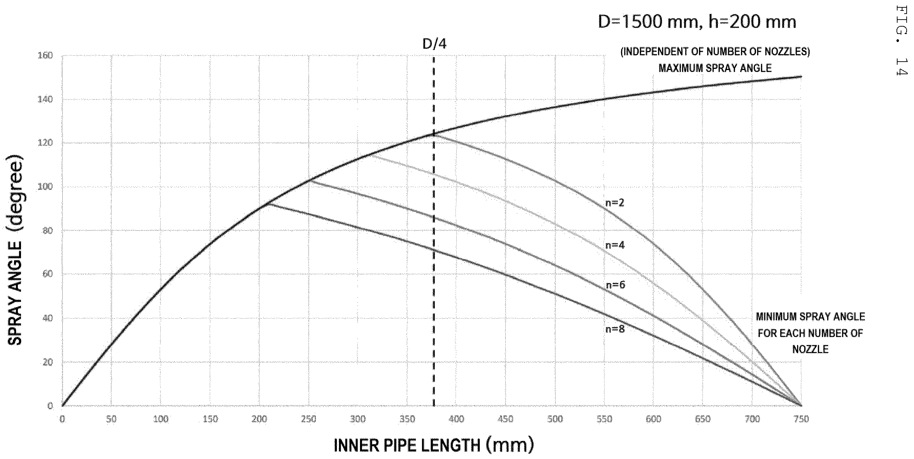

Meanwhile, FIGS. 13 and 14 are graphs illustrating a correlation between the minimum and maximum spray angles according to the inner pipe length in an embodiment, with the height h of the spray nozzle and the inner diameter (D) of the column fixed.

Specifically, FIG. 13 illustrates the maximum spray angle and minimum spray angle of the spray nozzle when the feed supply unit having four (n) spray nozzles is installed at a height of 200 mm in the feed stage A of a cylindrical column having an inner diameter of 1,500 mm. Referring to FIG. 13, when the length of the inner pipe is below a certain level, the maximum spray angle and the minimum spray angle of the spray nozzle are the same, but the length of the inner pipe is equal to or more than the certain level, the gap between the maximum spray angle and the minimum spray angle gradually increases. In addition, a region (a) of FIG. 13 is a case where the spray angle of the spray nozzle is larger than the maximum spray angle, and the feed spray region becomes excessively large, causing the feed within the column to flow to the column inner wall, which reduces the separation efficiency. A region (b) of FIG. 13 is a case where the spray angle of the spray nozzle is smaller than the minimum spray angle, and the area of the maximum spray region according to the length of the inner pipe cannot be achieved, so the feed within the column may be distributed inefficiently. A region (c) of FIG. 13 is a case where the spray angle of the spray nozzle satisfies the angle between the minimum spray angle and the maximum spray angle, the feed spray regions overlap and are efficiently distributed, and the feed does not flow to the column inner wall. Therefore, the inner pipe length corresponding to the region (c) can be set according to the spray angle of the commercial nozzle.

Specifically, FIG. 14 illustrates the maximum spray angle and minimum spray angle for each number of spray nozzles (n=2, 4, 6, and 8) when the feed supply unit is installed at a height h of 200 mm from the lower tray 210a in the feed stage of a cylindrical column having an inner diameter D of 1,500 mm.

Referring to FIG. 14, in a state where the height h of the spray nozzle and the inner diameter D of the column are fixed, the maximum spray angle of the spray nozzle is independent of the number n of spray nozzles, but the minimum spray angle is affected by the number (n) of nozzles, and accordingly, it can be confirmed that the length of the inner pipe, which becomes a branch point where the maximum spray angle and minimum spray angle of the spray nozzle change, also changes according to the number of spray nozzles. In addition, as the number of spray nozzles increases, the length of the inner pipe for optimal feed distribution can become shorter. However, the length (that is the depth of the spray nozzle) of the inner pipe can be affected by the diameter of the column and the height of the spray nozzle in addition to the number of spray nozzles. For example, when using 2 to 8 spray nozzles with the height h of the feed supply unit and the inner diameter D of the column fixed, it may be desirable for the length 1 of the inner pipe to be about 0.15D to 0.35D (that is, 0.15 to 0.35 times the inner diameter D of the column) in terms of optimal feed distribution.

Meanwhile, the separation device according to an embodiment of the present disclosure may further include a lower discharge port 400 provided at the bottom of the cylindrical column 100 to discharge a liquid stream and an upper discharge port 500 provided at the top of the cylindrical column to discharge a gaseous stream.

The raw material supplied into the cylindrical column 100 undergoes a separation process through continuous gas-liquid contact at each stage of the column, and the relatively light low-boiling-point component rises in a vapor state and is discharged through the upper discharge port 500 provided at the top, and the relatively heavy high-boiling-point component descends in a condensed liquid state and can be discharged through the lower discharge port 400 provided at the bottom. For example, when a feed containing acrylic acid is supplied, the acrylic acid can be discharged through the lower discharge port 400, but is not limited thereto.

In the dual flow tray 210 of each stage, the gaseous stream rising upward and the liquid stream descending downward come into contact with each other to transfer heat and mass, and as a result, a portion of the high-boiling-point component is condensed and flows down to the bottom, and the uncondensed vapor continues to rise to the top, and the process is continuously performed.

As described above, the reactive monomer separation device according to the present disclosure is described and illustrated in the drawings, but the description and illustration of the drawings describe and illustrate only the core components for understanding the present disclosure, and in addition to the processes and devices described and illustrated in the drawings, processes and devices not described and illustrated separately can be appropriately applied and utilized to implement the reactive monomer separation device according to the present disclosure.

In the above, exemplary embodiments of the present disclosure have been described, but the present disclosure is not limited thereto, and those skilled in the art will understand that various changes and modifications are possible within the scope of the claims described below.

[Description of symbols]

| | | | |
|---|---|---|---|
| 100: | cylindrical column | 200: | stage |
| 210: | dual flow tray | 215: | hole |
| 300: | feed supply unit | 310: | feed transfer pipe |
| 320: | inner pipe | 330: | spray nozzle |
| 400: | lower discharge port | 500: | upper discharge port |

**Claims**

1. A reactive monomer separation device, comprising:

    a cylindrical column;
    a plurality of dual flow trays provided inside the cylindrical column to partition a plurality of stages; and
    a feed supply unit provided in one of the plurality of stages to supply a raw material including a liquid reactive monomer,
    wherein the feed supply unit includes:

        a circular feed transfer pipe provided along an inner wall of the cylindrical column,
        one or more inner pipes extending in a direction of a central axis from the circular feed transfer pipe, and
        a spray nozzle provided at each end of each of the one or more inner pipes, and
        a spray angle ($\theta$) of the spray nozzle is equal to or less than a maximum spray angle defined by the following Equation (1):

$$\text{Equation (1): maximum spray angle} = 2 \times \tan^{-1}(l/h)$$

        wherein in the Equation (1), l is a length of the inner pipe, and h is a height from a lower dual flow tray of the stage equipped with the feed supply unit to the feed supply unit.

2. The reactive monomer separation device of claim 1, wherein the spray angle ($\theta$) of the spray nozzle is equal to or more than a minimum spray angle defined by the following Equation (2):

    minimum spray angle = $2 \times \tan^{-1} (\min[\{(D/2)\text{-}l\} \times \sin(\pi/n), l]/h)$          Equation (2):

    wherein in the Equation (2), D is an inner diameter of the cylindrical column, 1 is the length of the inner pipe, n is the number of spray nozzles, and h is the height from the lower dual flow tray of the stage equipped with the feed supply unit to the feed supply unit.

3. The reactive monomer separation device of claim 1, wherein when an inner diameter of the cylindrical column is D, the length l of the one or more inner pipes is more than 0 mm and less than 0.5D.

4. The reactive monomer separation device of claim 1, wherein the number of inner pipes is 2 to 10.

5. The reactive monomer separation device of claim 4, wherein number of the spray nozzles corresponds 1:1 to the number of the inner pipes.

6. The reactive monomer separation device of claim 4, wherein the inner pipes are provided spaced apart from each other at equal intervals.

7. The reactive monomer separation device of claim 1, wherein the feed transfer pipe is spaced apart between the lower dual flow tray and an upper dual flow tray in the stage in which the feed supply unit is provided.

8. The reactive monomer separation device of claim 7, wherein the feed supply unit is provided at a position closer to the upper dual flow tray than to the lower dual flow tray.

9. The reactive monomer separation device of claim 1, wherein the reactive monomer includes (meth)acrylic acid, acrylic acid, methyl acrylate, 1,3-butadiene, or a mixture thereof.

10. The reactive monomer separation device of claim 1, further comprising:

   a lower discharge port provided at a bottom of the cylindrical column for discharging a liquid stream; and
   an upper discharge port provided at a top of the cylindrical column for discharging a gaseous stream.

FIG. 1

FIG. 2

FIG. 3

FIG. 4

LIQUID
LEVEL

h

FIG. 5

LIQUID
LEVEL

h

FIG. 6

FIG. 7

FIG. 8

FIG. 9

FIG. 10

FIG. 11

FIG. 12

FIG. 13

FIG. 14

## INTERNATIONAL SEARCH REPORT

| International application No. |
|---|
| **PCT/KR2024/008754** |

| **A.** | **CLASSIFICATION OF SUBJECT MATTER** |
|---|---|

**B01D 3/32**(2006.01)i; **B01D 3/22**(2006.01)i; **B01D 3/42**(2006.01)i; **C07C 51/44**(2006.01)i; **C07C 67/54**(2006.01)i; **C07C 7/04**(2006.01)i; **C07C 57/04**(2006.01)i; **C07C 69/54**(2006.01)i; **C07C 11/167**(2006.01)i

According to International Patent Classification (IPC) or to both national classification and IPC

| **B.** | **FIELDS SEARCHED** |
|---|---|

Minimum documentation searched (classification system followed by classification symbols)

B01D 3/32(2006.01); B01D 3/00(2006.01); B01D 53/14(2006.01); B01D 53/48(2006.01); B01J 4/00(2006.01); B08B 9/093(2006.01)

Documentation searched other than minimum documentation to the extent that such documents are included in the fields searched

Korean utility models and applications for utility models: IPC as above
Japanese utility models and applications for utility models: IPC as above

Electronic data base consulted during the international search (name of data base and, where practicable, search terms used)

eKOMPASS (KIPO internal) & keywords: 듀얼플로우트레이(dual flow tray), 증류(distillation), 장치(apparatus), 노즐(nozzle), 배관(pipe), 모노머(monomer)

| **C.** | **DOCUMENTS CONSIDERED TO BE RELEVANT** |
|---|---|

| Category* | Citation of document, with indication, where appropriate, of the relevant passages | Relevant to claim No. |
|---|---|---|
| A | US 2020-0179889 A1 (ARKEMA FRANCE) 11 June 2020 (2020-06-11)<br>See claims 1, 5 and 8. | 1-10 |
| A | CN 108025220 A (BASF SE) 11 May 2018 (2018-05-11)<br>See claims 1 and 2. | 1-10 |
| A | KR 10-2009-0017131 A (AMTPACIFIC CO., LTD.) 18 February 2009 (2009-02-18)<br>See claim 1. | 1-10 |
| A | US 2004-0249198 A1 (THIEL, Joachim et al.) 09 December 2004 (2004-12-09)<br>See claims 11 and 20. | 1-10 |
| A | KR 10-2002-0011774 A (ACTIMAG CO. et al.) 09 February 2002 (2002-02-09)<br>See claim 1. | 1-10 |

☐ Further documents are listed in the continuation of Box C.  ☑ See patent family annex.

| | | | |
|---|---|---|---|
| * | Special categories of cited documents: | "T" | later document published after the international filing date or priority date and not in conflict with the application but cited to understand the principle or theory underlying the invention |
| "A" | document defining the general state of the art which is not considered to be of particular relevance | | |
| "D" | document cited by the applicant in the international application | "X" | document of particular relevance; the claimed invention cannot be considered novel or cannot be considered to involve an inventive step when the document is taken alone |
| "E" | earlier application or patent but published on or after the international filing date | | |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
|---|---|
| **25 September 2024** | **26 September 2024** |

| Name and mailing address of the ISA/KR | Authorized officer |
|---|---|
| **Korean Intellectual Property Office**<br>**Government Complex-Daejeon Building 4, 189 Cheongsa-ro, Seo-gu, Daejeon 35208** | |
| Facsimile No. **+82-42-481-8578** | Telephone No. |

Form PCT/ISA/210 (second sheet) (July 2022)

# EP 4 681 794 A1

## INTERNATIONAL SEARCH REPORT
### Information on patent family members

International application No.

**PCT/KR2024/008754**

| Patent document cited in search report | | | Publication date (day/month/year) | Patent family member(s) | | | Publication date (day/month/year) |
|---|---|---|---|---|---|---|---|
| US | 2020-0179889 | A1 | 11 June 2020 | BR | 112016000817 | A2 | 25 July 2017 |
| | | | | BR | 112016000817 | B1 | 09 November 2021 |
| | | | | CN | 105377412 | A | 02 March 2016 |
| | | | | CN | 105377412 | B | 04 December 2018 |
| | | | | EP | 3024564 | A1 | 01 June 2016 |
| | | | | EP | 3024564 | B1 | 08 July 2020 |
| | | | | FR | 3008899 | A1 | 30 January 2015 |
| | | | | FR | 3008899 | B1 | 30 January 2015 |
| | | | | JP | 2016-527240 | A | 08 September 2016 |
| | | | | JP | 6827807 | B2 | 10 February 2021 |
| | | | | KR | 10-2016-0034935 | A | 30 March 2016 |
| | | | | KR | 10-2187726 | B1 | 07 December 2020 |
| | | | | MX | 2016000771 | A | 27 April 2016 |
| | | | | SG | 11201600553 | A | 26 February 2016 |
| | | | | US | 10596537 | B2 | 24 March 2020 |
| | | | | US | 10919015 | B2 | 16 February 2021 |
| | | | | US | 2016-0175796 | A1 | 23 June 2016 |
| | | | | WO | 2015-011048 | A1 | 29 January 2015 |
| | | | | ZA | 201508870 | B | 29 November 2017 |
| CN | 108025220 | A | 11 May 2018 | BR | 112018000793 | A2 | 04 September 2018 |
| | | | | BR | 112018000793 | B1 | 03 March 2022 |
| | | | | CN | 108025220 | B | 30 March 2021 |
| | | | | DE | 102015213493 | A1 | 15 September 2016 |
| | | | | EP | 3325122 | A1 | 30 May 2018 |
| | | | | EP | 3325122 | B1 | 05 February 2020 |
| | | | | US | 2017-0014730 | A1 | 19 January 2017 |
| | | | | WO | 2017-012855 | A1 | 26 January 2017 |
| KR | 10-2009-0017131 | A | 18 February 2009 | | None | | |
| US | 2004-0249198 | A1 | 09 December 2004 | DE | 10156988 | A1 | 28 May 2003 |
| | | | | DE | 10295317 | B4 | 28 March 2013 |
| | | | | DE | 10295317 | D2 | 09 September 2004 |
| | | | | JP | 2005-509512 | A | 14 April 2005 |
| | | | | JP | 2005-509512 | T | 14 April 2005 |
| | | | | JP | 4116558 | B2 | 09 July 2008 |
| | | | | US | 7306204 | B2 | 11 December 2007 |
| | | | | WO | 03-043712 | A1 | 30 May 2003 |
| | | | | WO | 0304-3712 | A8 | 07 August 2003 |
| KR | 10-2002-0011774 | A | 09 February 2002 | KR | 10-0375605 | B1 | 10 March 2003 |

Form PCT/ISA/210 (patent family annex) (July 2022)

REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- KR 1020230124652